Europäisches Patentamt

European Patent Office

Office européen des brevets

(11) Publication number: **0 237 021**
**A1**

## (12) EUROPEAN PATENT APPLICATION

(21) Application number: **87103408.8**

(22) Date of filing: **10.03.87**

(51) Int. Cl.³: **A 61 B 17/11**

(30) Priority: **11.03.86 US 838535**

(43) Date of publication of application:
**16.09.87 Bulletin 87/38**

(84) Designated Contracting States:
**BE DE FR GB IT**

(71) Applicant: **RADTECH, INC.**
**2610 San Mateo, N.E., Suite D**
**Albuquerque New Mexico 87110(US)**

(72) Inventor: **Somers, John W.**
**305 Solano NE**
**Albuquerque New Mexico 87108(US)**

(72) Inventor: **Van Nieuwenborg, Bernardus W.**
**3114 Moon, NE**
**Albuquerque New Mexico 87111(US)**

(74) Representative: **Baillie, Iain Cameron et al,**
**c/o Ladas & Parry Isartorplatz 5**
**D-8000 München 2(DE)**

(54) Stem assisted retrievable vascular stent.

(57) A removable surgical stent assisted by a permanently positioned retrieval stem (2) for intraluminal placement in small vessels for repair thereof by anastomosis. Several embodiments are described which have some or all of the following features: great flexibility about the approximate location of the attachment of the retrieval stem (2), compact folded geometry of the stent body (1) about the approximate location of attachment of the retrieval stem (2), freedom from interference of the surgical stent in the suturing process, vessel support capability, and easy insertion of the surgical stent into the vessels to be repaired.

FIG. 1.

## STEM ASSISTED RETRIEVABLE VASCULAR STENT

### BACKGROUND OF THE INVENTION

The present invention is related generally to stents and more particularly to retrievable intraluminal vascular stents for small vessel repair by anastomosis.

Stents have been used for relief of tracheobronchial obstruction and hepatic stricture, for ear ventilation and packing after surgery, for drainage tubes, and for heart valve protheses, among other uses in the medical arts, as well as for small vessel repair. It is for this latter purpose that the retrievable stent of the present invention is directed.

It is well-known that one of the most important factors responsible for the occlusion of small vessels is the improper apposition of cut vessel edges with disruption of the media of the vessel wall resulting from the improper positioning of sutures. A solution to this problem is the use of silastic stents placed intraluminally over which the vessel is anastomosed to assist in the proper alignment of vessel edges and proper positioning of sutures. A stent suitable for such purpose is described in "An Intraluminal Silastic Stent for Small Vessel Repair," by R. R. Schenck and G. H. Derman, Orthopedic Clinics of North America 8, 265 (1977). Therein the authors teach a 3 by 0.5 mm. stent fashioned from firm medical grade silicone rubber in a dumbbell shape for placement intraluminally in microvessels. A 10-0 suture was tied to the center of the stent to assist in its removal just prior to the completion of the anastomosis. However, the surgeon or his support staff must remember to attach a suture to the stent prior to the surgical procedure in order to ready the stent for use. Moreover, the attached suture may become confused with other sutures in complicated

-2-

operations.  Clearly, an integral retrieval assistance tab or stem would relieve the surgical team of these concerns.

<u>SUMMARY OF THE INVENTION</u>

Consequently, the present invention provides a readily removable intraluminal stent for small vessel repair by anastomosis without a surgeon or his or her assitants having to attach the means for its removal thereto.

The invention also provides an easily identifiable device in the surgical environment intraluminal stent for small vessel repair.

According to one aspect of the invention, there is provided a removable surgical stent for anastomosis of small vessels, the stent comprising in combination a generally cylindrical stent body having a first end and a second end, and a retrieval stem disposed in a substantially radial direction from an attachment location substantially on the surface of the stent body approximately midway between the first end and the second end of the stent body.

The device of this invention includes a generally cylindrically shaped stent fabricated from biocompatible material having a significantly stiff response to axial and longitudinal compression, while permitting the stent to be easily bent about its mid-section for ready removal from partially sutured vessels, and having an integral or permanently attached tab or stem located approximately equally distant from the ends of the stent body and disposed in an approximately radial direction from the axis thereof for assisting in the removal of the stent from partially sutured vessels.  Preferably, the stent body is tapered at both ends to facilitate insertion into vessels. It is also preferred that the stent body have a notch located opposite the location of the stem in order to improve the bending of the stent body for ease of removal from partially sutured vessels. Preferably also, the stent body and stem are reinforced by strands located inside of the material from which the stent and stem are fabricated, a first strand running from approximately one end of the body of the stent into the stem for substantially the length thereof, and a second strand running from approximately the second end of the body of the stent into the stem for substantially the length thereof. The use of such strands, which may be fabricated from wire or other high tensile strength materials such as certain polymeric compositions, in the reinforcement of the attachment location of the retrieval stem to the stent body prevents separation thereof during periods of severe mechanical stress thereon and permits the notch to be cut deeply into the stent body in order to provide maximum flexibility and a compact folded geometry essential for easy removal from partially sutured vessels through the smallest possible aperture therein. The present stent may be colored to increase its visual contrast relative to the tissues and fluids present in the surgical environment.

In a further aspect of the present invention, the device hereof includes a generally cylindrically shaped stent fabricated from biocompatible material having a significantly stiff response to axial and longitudinal compression while permitting the stent to be easily bent about its mid-section for ready removal from partially sutured vessels, the stent body having a circumferential notch about the approximate mid-section thereof to improve the ease of bending of the stent about its mid-section and for permitting the suturing needle to pass from one of the vessels undergoing anastomosis to the other unimpeded by the stent body, and having an integral

-4-

or permanently attached tab or stem located approximately equally distant from the ends of the stent body and disposed in an approximately radial direction from the axis thereof for assisting in the removal of the stent from partially sutured vessels. Preferably, the stent body is tapered at both ends to facilitate insertion into vessels, but that a substantially untapered portion remains on the cylindrical surface of the stent body between the circumferential notch and the tapered portion of the stent body to support the vessels undergoing anastomosis. Preferably also, the stent body and stem are reinforced with strands located inside of the material from which the stent and stem are fabricated, a first strand running from approximately one end of the body of the stent into the stem for substantially the length thereof, and a second strand running from approximately the second end of the body of the stent into the stem for substantially the length thereof. The use of such strands, which may be fabricated from wire or other high tensile strength materials such as certain polymeric compositions, in the reinforcement of the attachment location of the retrieval stem to the stent body prevents separation thereof during periods of severe mechanical stress thereon and permits the circumferential notch to be cut deeply into the stent body in order to provide maximum flexibility and a compact folded geometry essential for easy removal from partially sutured vessels through the smallest possible aperture therein. The present stent may be colored to increase its visual contrast relative to the tissues and fluids present in the surgical environment. For some applications, it is preferred that the stent body be asymmetrically shaped such that the two halves fit more closely together when folded during the removal process. In particular, the approximately cylindrical, substantially untapered vessel support section of one half of the stent body preferably nests into a depression adapted to receive

-5-

it in the other half of the stent body in the vessel support section thereof.

In yet a further aspect of the present invention, the device hereof includes a generally cylindrically shaped stent fabricated from biocompatible material having a significantly stiff response to axial and longitudinal compression while permitting the stent to be easily bent about its body axis for ready removal from partially sutured vessels, the stent body having a circumferential notch located at a position offset from the approximate mid-section thereof to improve the ease of bending of the stent about the location of the circumferential notch and for permitting the suturing needle to pass from one of the vessels undergoing anastomosis to the other unimpeded by the stent body, and having an integral or permanently attached tab or stem located within the circumferential notch at approximately the position of minimum diameter thereof and disposed in an approximately radial direction from the axis of the stent body for assisting in the removal of the stent from partially sutured vessels. It is preferred that the circumferential notch be asymmetric in length along the axis of the stent body in order that the shorter leg of the stent body divided by the circumferential notch may substantially tuck into the relieved portion of the longer leg provided by the circumferential notch thereby providing a more compact folded geometry when the stent of the present invention is removed from partially anastomosed vessels. Preferably, the stent body is tapered at both ends to facilitate insertion into vessels, but that an untapered portion remains on the cylindrical surface of the stent body between the circumferential notch and the tapered portion of the stent body to support the vessels undergoing anastomosis. Preferably also, the stent body and stem are reinforced with strands located inside of the material from which the stent and stem are fabricated, a first strand running from approximately one end of the body of the stent into the stem for substantially the length thereof, and a second strand running from approximately the second end of the body of the stent into the stem for substantially the length thereof. The use of such strands, which may be fabricated from wire or other high tensile strength materials such as certain polymeric compositions, in the reinforcement of the attachment location of the retrieval stem to the stent body prevents separation thereof during periods of severe mechanical stress thereon and permits the circumferential notch to be cut deeply into the stent body in order to provide maximum flexibility and a compact folded geometry essential for easy removal from partially sutured vessels through the smallest possible aperture therein. The present stent may be colored to increase its visual contrast relative to the tissues and fluids present in the surgical environment.

Benefits and advantages of the present invention include the ready removal of the stent of the invention from partially sutured vessels without the surgical team having to attach a suture to the stent in preparation for its later removal, and the easy identification of the stent removal stem among the sutures present in the environment of use of the stent. The deeply circumferentially notched body of the buried wire reinforced embodiment of the stent of our invention permits great flexibility thereof about the minimum diameter location of the circumferential notch, and the asymmetrical notch embodiment gives rise to an especially compact folded geometry of the stent for its removal from partially sutured vessels.

BRIEF DESCRIPTION OF THE DRAWINGS

The accompanying drawings, which are incorporated in and form a part of the specification, illustrate three embodiments of the present invention and, together with the description, serve to explain the principles of the invention. Similar or identical structure described depicted therein are identified by the same callout numbers throughout. In the drawings:

Figure 1 is a schematic representation of the stent of the present invention in its simplest form. Shown is the basic cylindrical stent body with the retrieval stem which is formed integrally with the stent body radially disposed thereto.

Figure 2 is a schematic representation of the stent of our invention where the stent body has pointed ends to facilitate insertion thereof into vessels.

Figure 3 is a schematic representation of the device of the present invention showing a stent body having a notch located opposite to the location of the retrieval stem for the purpose of increasing the flexibility of the stent about a region near the center of the stent body and to provide a compact folded geometry of the stent body in order to facilitate its removal with the aid of the retrieval stem from partially sutured vessels.

Figure 4 is a schematic representation of our stent showing two buried reinforcement strands for the purpose of strengthening the attachment location of the retrieval stem against the possibility of separation from the stent body under conditions of severe mechanical stress, while not interfering appreciably with the flexibility of the stent about its midpoint. In fact, the use of the reinforcement strands permits the notch to be cut more deeply into the stent body.

Figure 5 is a schematic representation of the stent described in Fig. 4 hereof in its bent position during removal thereof from partially sutured vessels.

Figure 6 is a schematic representation of the stent of our invention showing the symmetric circumferential

-8-

notch embodiment thereof and the vessel supporting cylindrical portions of the stent body. It is to be noticed that the stent retrieval stem may be flat or cylindrical in shape.

Figure 7 is a schematic representation of the stent of our invention showing the symmetrical circumferential notch embodiment thereof with the stent body being shaped in such a manner that a closer folded geometry results while maintaining the essential features of the vessel supporting surfaces of the stent body.

Figure 8 is a schematic representation of the stent shown in Fig. 7 hereof showing the more compact folded geometry thereof than that shown in Fig. 5 hereof.

Fig. 9 is a schematic representation of the asymmetric circumferentially notched embodiment of our invention.

Fig. 10 is a schematic representation of the stent shown in Fig. 9 hereof showing the folded configuration thereof.


DETAILED DESCRIPTION OF THE INVENTION

Briefly, the device of the present invention includes an improved stent design for intraluminal placement in small vessels to assist in surgical anastomoses. A retrieval stem integral with the stent body or permanently attached thereto and disposed in a generally radial direction from the axis of the approximately cylindrical stent body from an attachment location on the surface of the stent corresponding to the long dimension thereof is provided to assist in the removal of the stent body just prior to completion of a particular anastomosis procedure. The integral nature of the retrieval stem enables the surgical team to concentrate on other aspects of the surgical procedure without having to remember to attach

-9-

suture material to the stent to assist in its removal as taught by Schenck et al., supra. The retrieval stem can be integrally formed with the stent body or rigidly attached thereto if formed from materials other than that from which the stent body is formed. In one embodiment of the present invention, a deep notch is cut in the stent body opposite to the point of attachment of the retrieval stem in order to increase the flexibility of the stent about the center of the stent body thereby further facilitating its removal from partially sutured vessels. Reinforcement strands are provided inside of the stent and extending from the stent body into the retrieval stem to reduce the possibility of separation of the retrieval stem from the stent body at its point of attachment during severe mechanical stress, while permitting the notch to be made sufficiently deep to insure excellent flexibility and a compact geometry of the folded stent during removal. In another embodiment of our invention, a circumferential groove is cut in the stent body surface in the region of attachment of the retrieval stem in order to both improve the flexibility of the stent body during the removal process and to permit the surgical needle to more readily avoid the stent body during the anastomosis process. Certain shapes for the stent body have been found to create a more compact folded geometry which is useful in the removal of the stent from partially sutured vessels. Clearly, reinforcement strands of higher tensile strength material than the stent body may be employed to prevent detachment of the retrieval stem from the surface of the stent body when it is desirable to remove a significant amount of stent material in the formation of the circumferential notch in a similar manner to the simple notched embodiment described hereinabove. The entire stent is fabricated from firm biocompatible materials which may be tinted to increase the contrast thereof relative to

-10-

tissue and fluids present in the surgical environment.

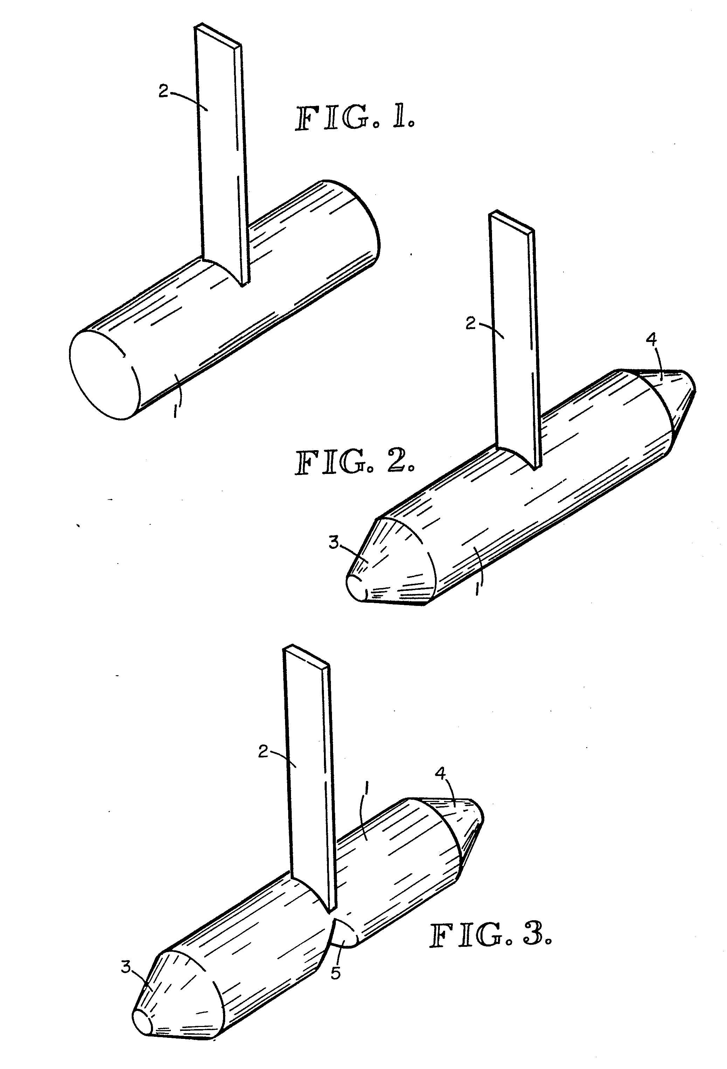

Reference will now be made in detail to the present preferred embodiments of the present invention, examples of which are illustrated in the accompanying drawings. Turning now to Fig. 1 hereof, the stent of the present invention is represented in its most general embodiment. A generally cylindrical stent body 1 having a first end and a second end has attached to its surface approximately at the midpoint between the first and second ends a retrieval stem 2 to assist in its removal from a partially sutured vessel. In its use, according to well-known medical procedure, the first end of the stent body is inserted into the end of a first appropriately prepared vessel, and the second end of the stent body inserted into the end of a second appropriately prepared vessel, the intent being to suture the two vessel ends together. The stitching process is incompletely performed in order to leave sufficient space for the removal of the assisting stent before the anastomosis is completed. The stent retrieval stem is simply grasped using a surgical forceps or other grasping tool to remove the stent of the present invention, and the suturing process completed.

Fig. 2 shows an embodiment of the present invention where the stent body 1 has tapered ends 3,4 for ease of insertion into a vessel end intended for anastomosis.

Fig. 3 shows an embodiment of the invention further including a deep notch 5 cut in the surface of the stent body opposite to the location of attachment of the retrieval stem 2. The notch serves two important functions in assisting in the removal of the stent just prior to the completion of the anastomosis procedure. First, it provides increased flexibility of the stent body in the direction opposite to the retrieval stem. Moreover, it enables the folded stent body to assume a more compact geometry during the removal process. This permits a more

-11-

complete closure of the vessels to be joined before the stent need be removed, with the consequent improvement in the quality and ease of performance of the anastomosis process.

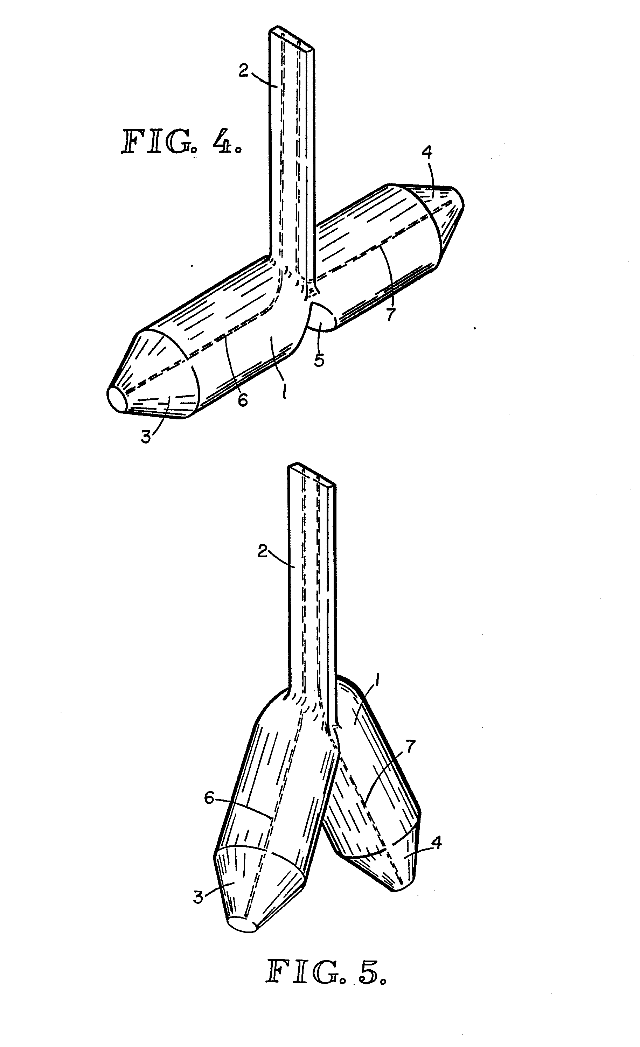

Fig. 4 shows a reinforced embodiment of the stent shown in Fig. 3 hereof. Strands 6,7 having greater tensile strength than the material of the stent body are buried in the stent material to prevent the possibility of separation of the retrieval stem from the stent body under the mechanical stress of its removal from a nearly anastomized vessel. In one of many possible reinforcing strand arrangements, a first strand 6 extends for a significant length into the one side of the stent body and is brought into the retrieval stem in the vicinity of the latter's attachment location to the stent body. A second reinforcing strand 7 extends for a significant lenth into the second side of the stent body and is similarly brought into the retrieval stem in the vicinity of the latter's attachment location to the stent body. The reinforcement of the stent/retrieval stem system according to the teachings of the present invention permits the notch 5 to be cut more deeply into the body of the stent thereby increasing its flexibility away from the direction of deployment of the retrieval stem, and decreasing the folded size of the stent body during the removal process.

Fig. 5 shows the stent described in Fig. 4 hereinabove in its folded configuration as would be the situation when the stent of the present invention is removed from a partially anastomosed pair of vessels.

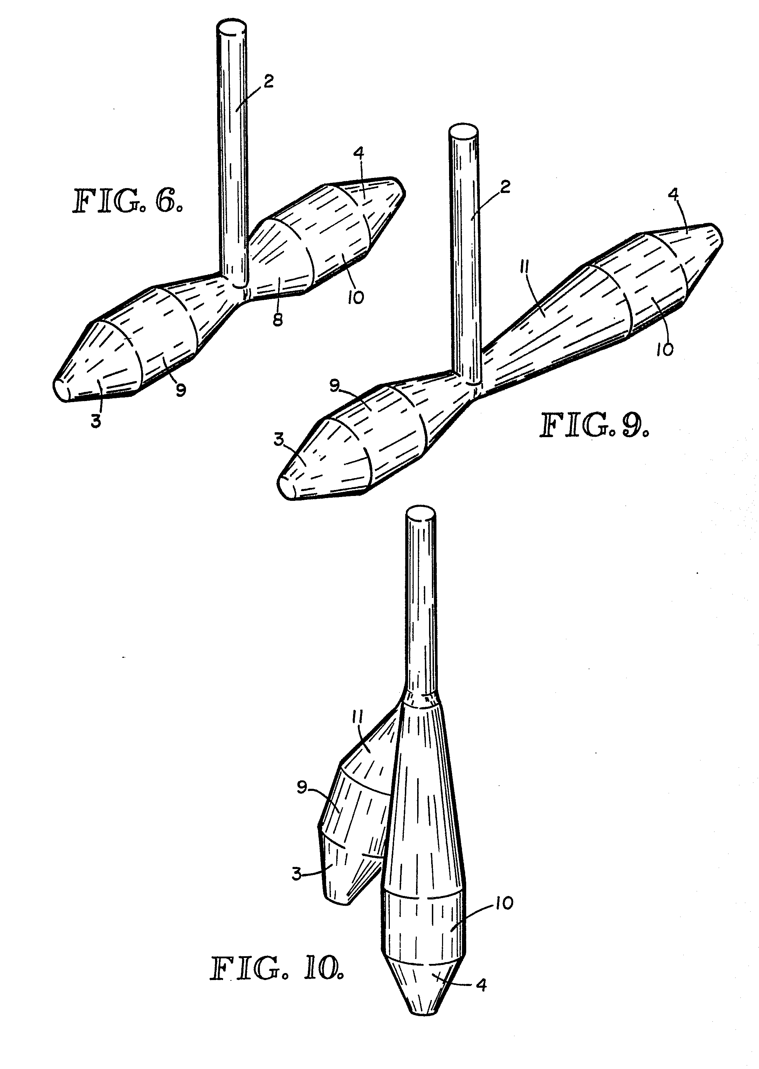

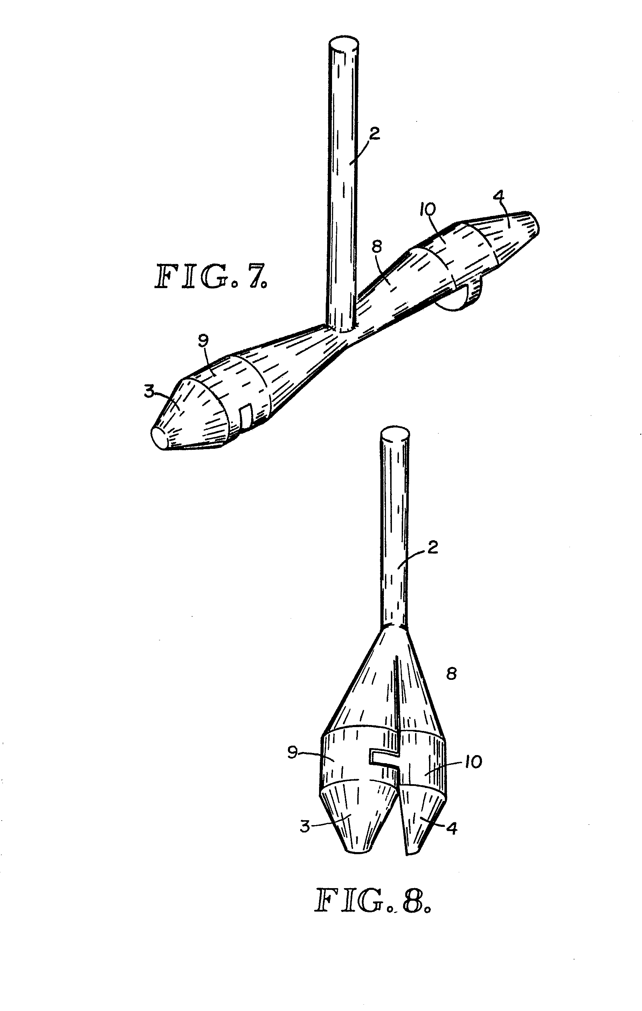

Fig. 6 shows one embodiment of our invention having a circumferential notch 8 symmetrically located relative to the ends of the stent body. Vessel supporting, approximately cylindrical portions 9,10 are formed on the stent body surface between the circumferential notch and the tapered ends of the stent body. The circumferential

-12-

notch 8 permits the surgical needle to enter and leave the vessels undergoing anastomosis without interference from the stent body, and allows increased flexibility of the stent body during the removal process than the embodiments of the present invention shown in Figs. 1 and 2. Figure 6 shows further that the retrieval stem 2 may have other than a flat geometry.

Figure 7 shows the embodiment of the stent of the present invention having a circumferential notch 8 symmetrically located relative to the ends of the stent body as described in Fig. 6 hereinabove and further having a shaped stent body such that vessel support surfaces 9,10 retain essentially the same function they had in Fig. 6, but are fashioned to be intermeshing on the side of the stent body away from the location of attachment of the retrieval stem such that the folded geometry shown in Fig. 8 is significantly smaller than stents designed without significant removal of stent body material in this portion of the stent body. Clearly many stent body shapes may be envisioned which accomplish this purpose.

Figures 9 and 10 hereof show that significant improvement in the compactness of the folded stent geometry is achievable by forming an asymmetrical circumferential groove 11 such that one end of the stent body nests into the region of the circumferential groove formed in the other end thereof. Clearly, many similar arrangements may be envisioned.

The foregoing description of four preferred embodiments of the present invention has been presented for purposes of illustration and description. It is not intended to be exhaustive or to limit the invention to the precise form disclosed, and many modifications and variations are possible in light of the above teaching. For example, although the preferred embodiment of the present stent has the retrieval stem integrally formed with

-13-

the stent body during the fabrication process, it may be desirable to fabricate the stem out of different materials than that of the stent body or having a different color therefrom, and to rigidly attach the stem to the stent body in a separate process. Moreover, the reinforcing strands might be fabricated from wire or polymeric material having higher tensile strength than the stent body depending upon choice of biocompatible stent and retrieval stem materials, since it is desirable that the reinforcing strands bond to these materials in order to reduce the possibility of separation of the stent body and the retrieval stem during the surgical procedure. Further, reinforcement strands may be included in the embodiments of our invention having the circumferential notch although none are explicitly shown in the accompanying figures. The embodiments were chosen and described in order to best explain the principles of the invention and its practical application to thereby enable others skilled in the medical art to best utilize the invention in various embodiments and with various modifications as are suited to the particular use contemplated. It is intended that the scope of the invention be defined by the claims appended hereto.

# CLAIMS

1. A removable surgical stent for anastomosis of small vessels, said stent comprising in combination a generally cylindrical stent body having a first end and a second end, and a retrieval stem disposed in a substantially radial direction from an attachment location substantially on the surface of said stent body approximately midway between the first end and the second end of said stent body.

2. The removable surgical stent as described in Claim 1, wherein said retrieval stem is formed integrally with said stent body.

3. The removable surgical stent as described in Claims 1 or 2, wherein said stent body is notched at a location approximately opposite the attachment location of said retrieval stem for increasing the flexibility of said stent body about the approximate midpoint between the first end and the second end thereof in order to improve the ease of removal thereof from partially anastomosed vessels.

4. The removable surgical stent as described in Claim 3, wherein reinforcement strands having a tensile strength greater than that for the material from which the surgical stent is fabricated are provided to strengthen the attachment location of said retrieval stem in order to permit the notch to be made deeper, thereby providing greater flexibility of said stent body about the approximate midpoint between the first end and the second end thereof and a more compact folded geometry when said stent body is removed from partially anastomosed vessels.

5. The removable surgical stent as described in Claim 4, wherein the first end and the second end of said stent body are tapered to enable the insertion thereof into vessels to be more readily achieved.

-15-

6.    The removable surgical stent as described in
Claim 5, wherein said stent body is fabricated from
biocompatible silastic materials.

7.    The removable surgical stent as described in
Claim 6, wherein said biocompatible silastic materials
include colored silastic materials for the purpose of
readily identifying said stent body and said retrieval
stem in the presence of tissue and fluids found in the
surgical environment.

8.    The removable surgical stent as described in
Claims 1 or 2, wherein said stent body has a substantially
symmetrical circumferential notch in the region of the
attachment location of said retrieval stem for reducing
the interference of the stent body with a surgical
needle utilized in the anastomosis process and for
increasing the flexibility of said stent body about the
approximate midpoint between the first end and the second
end thereof in order to improve the ease of removal
thereof from partially anastomosed vessels.

9.    The removable surgical stent as described in
Claim 8, wherein reinforcement strands having a tensile
strength greater than that for the material from which
the surgical stent is fabricated are provided to strengthen
the attachment location of said retrieval stem in order to
permit the circumferential notch to be made deeper, thereby
providing greater flexibility of said stent body about the
approximate midpoint between the first end and the second
end thereof and a more compact folded geometry when said
stent body is removed from a partially anastomosed vessel.

10.    The removable surgical stent as described in
Claim 9, wherein the first end and the second end of said
stent body are tapered to enable the insertion thereof
into vessels to be more readily achieved.

11.    The removable surgical stent as described in Claim 10, wherein a substantially cylindrical vessel support region on the surface of said stent body between the circumferential notch therein and each of the tapered ends thereof.

12. The removable surgical stent as described in Claim 11, wherein the side of said stent body opposite the direction of disposition of said retrieval stem is shaped such that the vessel support region on the side of said stent body opposite the direction of disposition of said retrieval stem formed on one side of said retrieval stem substantially fits into a receiving depression formed in the support region on the side of said stent body opposite the direction of disposition of said retrieval stem on the other side of said retrieval stem, whereby a more compact folded geometry for said stent body is achieved during the removal of the surgical stent from partially anastomosed vessels.

13. The removable surgical stent as described in Claim 12, wherein said stent body is fabricated from biocompatible silastic materials.

14. The removable surgical stent as described in Claim 13, wherein said biocompatible silastic materials include colored silastic materials for the purpose of readily identifying said stent body and said retrieval stem in the presence of tissue and fluids found in the surgical environment.

15. The removable surgical stent as described in Claims 1 or 2, wherein said stent body is divided into a short end and a long end by an asymmetrical circumferential notch in the region of the attachment location of said retrieval stem for reducing the interference of the stent body with a surgical needle utilized in the anastomosis process, for increasing the flexibility of said stent body about the approximate attachment location of said retrieval stem in order to improve the ease of removal

-17-

thereof from partially anastomosed vessels, and for enabling the shorter end of said retrieval stem to substantially fit into the circumferential notch portion of the larger end of said stent body in order to achieve a more compact folded geometry therefor when said stent body is folded during the removal thereof from partially anastomosed vessels.

16. The removable surgical stent as described in Claim 15, wherein reinforcement strands having a tensile strength greater than that for the material from which the surgical stent is fabricated are provided to strengthen the attachment location of said retrieval stem in order to permit the circumferential notch to be made deeper, thereby providing greater flexibility of said stent body about the approximate attachment location of said retrieval stem and a more compact folded geometry when said stent body is removed from a partially anastomosed vessel.

17. The removable surgical stent as described in Claim 16, wherein the first end and the second end of said stent body are tapered to enable the insertion thereof into vessels to be more readily achieved.

18. The removable surgical stent as described in Claim 17, wherein a substantially cylindrical vessel support region on the surface of said stent body between the asymmetrical circumferential notch therein and each of the tapered ends thereof.

19. The removable surgical stent as described in Claim 18, wherein said stent body is fabricated from biocompatible silastic materials.

20. The removable surgical stent as described in Claim 19, wherein said biocompatible silastic materials include colored silastic materials for the purpose of readily identifying said stent body and said retrieval stem in the presence of tissue and fluids found in the surgical environment.

1/4 FIG. 3 0237021

FIG. 1.

FIG. 2.

FIG. 3.

0237021

FIG. 4.

FIG. 5.

0237021

FIG. 6.

FIG. 9.

FIG. 10.

0237021

FIG. 7.

FIG. 8.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | FR-A-2 274 265  (VSESOJUZNY NAUCHNO-ISSLEDOVATELSKY INSTITUT KHIRURGICHESKOI APPARATURY I INSTRUMENTOV) <br> * page 4, lines 17-31;  page 10, lines  16-35;  page 13,  lines 25-29; page 19, lines 1-8; claims 2, 3; figures 16, 17 * | 1,3,6 | A 61 B  17/11 |
| | --- | | |
| A | US-A-4 483 339  (GILLIS) <br> * column 1, lines 8-22; claim  1; figures 3-5 * | 1,5 | |
| | --- | | |
| A,P | EP-A-0 183 372  (RAYCHEM CORP.) <br><br> * page 1, lines 1-5;  page 7, lines 15-22; page 8, lines 24-28; page 11, lines 11-17;  claims 1, 9, 12, 13; figures 3-5 * | 1,6,13 ,19 | |
| | | | TECHNICAL FIELDS SEARCHED (Int. Cl.4) |
| | --- | | |
| A | WO-A-8 503 858  (AMARASINGHE) <br> * page 6, line 22 - page 7, line 4; claim 1; figures 2-4 * | 1,5,6 | A 61 B  17/00 <br> A 61 B  19/00 <br> A 61 M  29/00 |
| | --- | | |
| A | FR-A-2 242 961  (MICHEAU) <br> * page 1, lines 1-6, 14-24;  figures 1, 2. * | 1,5 | |
| | --- | | |
| A | US-A-3 805 793  (WRIGHT) <br> * column  3, line 55 - column 4, line 13; claim 1; figures 4, 5 * | 1 | |
| | ----- | | |

The present search report has been drawn up for all claims

| Place of search | Date of completion of the search | Examiner |
|---|---|---|
| BERLIN | 02-06-1987 | MONNE E.M.B. |